# EUROPEAN PATENT APPLICATION

(11) **EP 4 506 024 A1**
(43) Date of publication of application: **12.02.2025**
(21) Application number: 23784345.3
(22) Date of filing: 07.04.2023
(51) Int. Cl.: A61M 5/142

(54) **INFUSION DEVICE, ASSEMBLY METHOD THEREFOR, AND USE THEREOF**

(30) Priority: 07.04.2022 CN 202210360314; 07.04.2022 CN 202210360312; 07.04.2022 CN 202210361379; 07.04.2022 CN 202210361394; 07.04.2022 CN 202210361391
(71) Applicant: MicroTech Medical (Hangzhou) Co., Ltd., Hangzhou, Zhejiang 311121 (CN)
(72) Inventor: SONG, Zhe, Hangzhou, Zhejiang 311121 (CN); CHEN, Ningning, Hangzhou, Zhejiang 311121 (CN)
(74) Representative: Keltie LLP
(86) International application number: PCT/CN2023/086805
(87) International publication number: WO 2023/193782

(57) **Abstract**

An infusion device, a means of assembling the infusion device, and a use. The infusion device comprises an infusion pump (40) and a liquid storage device (112) connected to the infusion pump (40). The liquid storage device (112) and the infusion pump (40) are L-shaped, and the infusion pump (40) is connected to the liquid storage device (112) by means of a slide rail. Said infusion device can be produced as modules, assembly and detachment are convenient, and said device has superior water resistance.

## Description

### Technical Field

The present invention relates to the technical field of medical instruments, and in particular to an infusion device, an assembly method therefor and use thereof.

### Background Art

An infusion device is a medical instrument that administrates a prescribed amount of a drug at a prescribed time, which achieves the supplementation of the drug, and can meet the treatment requirements of patients.

### Summary of the invention

The present invention proposes an infusion device which can be produced in a modular manner, is convenient to mount and dismount, and has excellent waterproof performance.

A first aspect of the present invention provides an infusion device, comprising an infusion pump (40) and a liquid storage device (112) connected to the infusion pump, characterized in that the liquid storage device (112) and the infusion pump are L-shaped, wherein the side surfaces of the liquid storage device (112) is composed of a first L-shaped side surface (1112) of the liquid storage device, a long side surface (1113) of the liquid storage device, a second L-shaped side surface (1114) of the liquid storage device, and a short side surface (1118) of the liquid storage device; the middle side surface (1116) of the liquid storage device is located between the long side surface (1113) of the liquid storage device and the short side surface (1118) of the liquid storage device; the bottom surface (1115) of the liquid storage device and the lower top surface (1117) of the liquid storage device are adjacent to the middle side surface (1116) of the liquid storage device; and the lower top surface (1117) of the liquid storage device is located between the bottom surface (1115) of the liquid storage device and the upper top surface (1111) of the liquid storage device; the side surfaces of the infusion pump (40) is composed of a first L-shaped side surface (1122) of the infusion pump, a long side surface (1123) of the infusion pump, a second L-shaped side surface (1124) of the infusion pump, and a short side surface (1128) of the infusion pump; the middle side surface (1126) of the infusion pump is located between the long side surface (123) of the infusion pump and the short side surface (1128) of the infusion pump; the upper top surface (1121) of the infusion pump and the low top surface (1127) of the infusion pump are adjacent to the middle side surface (1126) of the infusion pump; and the low top surface (1127) of the infusion pump is located between the upper top surface (1121) of the infusion pump and the bottom surface (1125) of the infusion pump.

Optionally, the infusion pump (40) and the liquid storage device (112) are connected by means of a slide rail.

Optionally, the middle side surface (1116) of the liquid storage device is provided with guide rails (115) perpendicular to the upper top surface (1111) of the liquid storage device, and the middle side surface (1126) of the infusion pump is provided with tracks (114) perpendicular to the bottom surface (1125) of the infusion pump; the guide rail (115) and the track (114) are engaged with each other so that they can move relative to each other; the infusion pump comprises an upper housing assembly (32) and a lower housing assembly (33);a first sealing member is provided between the upper housing assembly of the infusion pump and the lower housing assembly of the infusion pump; the upper housing assembly of the infusion pump and the lower housing assembly of the infusion pump are connected by means of a detachable connecting structure. Therefore, the infusion pump can be integrally disassembled, which is convenient to assemble and facilitates subsequent maintenance.

Optionally, the guide rail (115) extends from the bottom surface (1115) of the liquid storage device to the lower top surface (1117) of the liquid storage device, and the track (114) extends from the upper top surface (1121) of the infusion pump to the lower top surface (1127) of the infusion pump.

Optionally, the guide rail (115) has a first edge (532), and when the guide rail (115) slides relative to the track (114), the first edge (532) fits the track (114).

Optionally, there is an angle between the first edge (532) and the center line of the middle side surface (1116) of the liquid storage device, and the angle ranges from 0.5° to 5°.

Optionally, the end of the guide rail (115) that is close to the lower top surface (1117) of the liquid storage device is provided with a limiting member (531), which is located in a groove (533) between the guide rail (115) and the middle side surface (1116) of the liquid storage device.

Optionally, the track (114) is provided with a projecting portion (543) stretching inward, and the track (114) is provided with a second edge (542).When the track (114) slides relative to the guide rail (115), the second edge (542) fits the guide rail (115).

Optionally, there is an angle between the second edge (542) and the center line of the middle side surface (1126) of the infusion pump, and the angle ranges from 0.5° to 5°.

Optionally, the end of the track (114) that is close to the lower top surface (1127) of the infusion pump is provided with a clamping member, which clamps the track (114) and the guide rail (115) at the bottom of the track (114).

Optionally, the clamping member is a protrusion (541) arranged on the track (114).

Optionally, the first sealing member is a first sealing member (117);the upper housing assembly of the infusion pump and the lower housing assembly of the infusion pump are provided with an upper housing (11) of the infusion pump and a lower housing (19) of the infusion pump respectively, and the first sealing member (117) is located on a butt-joined surface between the lower housing (19) of the infusion pump and the upper housing (11) of the infusion pump; the lower housing (19) of the infusion pump and the upper housing (11) of the infusion pump are connected by means of screws.

Optionally, the first sealing member is integrally injection-molded with the lower housing (19) of the infusion pump.

Optionally, the infusion device further comprises a bottom plate (210).The infusion pump(40) is connected to the liquid storage device (112) by means of a first snap-fit structure, wherein the portion of the first snap-fit structure located in the liquid storage device (112) can move during the assembly process of the liquid storage device with the infusion pump and is restored and maintained in a fixed position under the action of a first spring. The infusion pump(40) is connected to the liquid storage device (112), and then is connected to the bottom plate (210) by means of a second snap-fit structure, wherein the portion of the second snap-fit structure located in the liquid storage device (112) can move during the assembly process with the bottom plate, and is restored and maintained in a fixed position under the action of a second spring. The first spring and the second spring are the same spring (26). The first snap-fit structure comprises a hook (116) arranged on the upper top surface (1121) of the infusion pump and a bayonet (1132) arranged on the lower top surface (1117) of the liquid storage device, wherein the hook (116) corresponds to the bayonet (1132);
the inner side of the bayonet (1132) is provided with a pin (1130) for snapping with the hook (116), and the pin (1130) is connected to a side push button (1131) arranged on a shell of the liquid storage device.

Optionally, the pin (1130) is connected to a connecting rod (2383), and the connecting rod (2383) is connected to a side push button (1131) arranged on the shell (240) of the liquid storage device.

Optionally, the inner side of the side push button (1131) is provided with a spring (26) abutting against the side push button (1131).

Optionally, one side of the pin (1130) is provided with a slope (23821). When the hook (116) enters the bayonet (1132), the hook (116) presses against the slope (23821), so that the pin (1130) moves toward one side, thereby squeezing the spring (26).

Optionally, the bottom plate (210) is provided with a bottom plate buckle (237). The shell (240) of the liquid storage device is provided with a buckle inlet (28), the inner side of which is provided with a top buckle (236). The top buckle (236) is provided with a claw (2363) for snapping with the bottom plate buckle (237). The claw (2363) is connected to a top push button (2361) arranged on the shell (240) of the liquid storage device by means of a connecting member. Therefore, the infusion pump and the liquid storage device of the present invention are convenient and quick to assemble, easy to disassemble, firm and reliable, and occupy a small space.

Optionally, the connecting member is a spring frame (2362). The spring frame (2362) is opposite to the inner side of the side push button (1131). The spring (26) is located between the spring frame (2362) and the side push button (1131).

Optionally, one side of the claw (2363) is provided with an inclined surface (23631). When the bottom plate buckle (237) enters the buckle inlet (28), the bottom plate buckle (237) presses against the inclined surface (23631), so that the claw (2363) moves toward one side, thereby squeezing the spring (26).

Optionally, a pump bottom groove (235) is provided at the end on the side of the infusion pump (40) that is close to the bottom plate (210),and a bottom plate protrusion (239) is provided at a corresponding position on the bottom plate (210), so that the bottom plate protrusion (239) can be inserted into the pump bottom groove (235) and can rotate relatively in the pump bottom groove (235).

Optionally, the bottom plate buckle (237) and the bottom plate protrusion (239) are located at two ends of the bottom plate (210) respectively.

Optionally, the upper housing assembly of the infusion pump further comprises an antenna (12), a buzzer (113), a printed circuit board (13), a rechargeable battery (14), a gearbox assembly (15) and a transmission assembly, wherein the upper housing (11) of the infusion pump is connected to all of the antenna (12), the printed circuit board (13), the rechargeable battery (14), the gearbox assembly (15) and the transmission assembly; the buzzer (113) is attached to the inner side of the upper housing (11) of the infusion pump and is connected to the printed circuit board (13).

Optionally, the printed circuit board (36) is arranged on the side of the upper housing assembly (32) of the infusion pump that is adjacent to the lower housing assembly (33) of the infusion pump.

Optionally, the rechargeable battery (14) is provided to be connected to the printed circuit board (36).

Optionally, the transmission assembly comprises a sleeve (124), a screw rod (127), a pressure sensor (130), a nut push rod (126), and a contact switch (128), wherein the sleeve (124) is located on the upper housing (11) of the infusion pump; the nut push rod (126) is arranged in the sleeve (124), the screw rod (127) is located at the center of the nut push rod (126), and the screw rod (127) is threadedly connected to the nut push rod (126); the gearbox assembly (15) is connected to the screw rod (127); the pressure sensor (130) is arranged at the bottom of the screw rod (127); the contact switch (128) is arranged at the bottom of the stroke of the nut push rod (126).

Optionally, a piston (111) is provided in the liquid storage device (112). An infusion needle (131) is connected to a cavity above the piston (111) by means of a pipeline. The piston (111) is opposite to the nut push rod (126) and is arranged at the top of the nut push rod (126).

Optionally, the piston (111) is arranged on the inner side of the opening of the liquid storage device (112). The piston (111) is connected to the inner wall (411) on the inner side of the opening in the bottom surface of the liquid storage device by means of a fourth sealing member (49).

Optionally, the fourth sealing member (49) is an O-ring arranged on the outer side of the piston (111).

Optionally, the liquid storage device is provided with an opening, and the sleeve (124) is sealedly connected to the inner wall (411) on the inner side of the opening in the bottom surface of the liquid storage device.

Optionally, the outer side of the sleeve (124) is provided with a second sealing member (45), which is a sealing ring. The sealing connection between the infusion pump and the inner wall (411) of the liquid storage device is achieved by means of the second sealing member (45).

Optionally, the upper housing (11) of the infusion pump is provided with a sleeve mounting hole (416), and a third sealing member (48) is provided between the sleeve (124) and the upper housing (11) of the infusion pump.

Optionally, a recess (461) is provided at a portion where the sleeve (124) is connected to the sleeve mounting hole (416), and the third sealing member (48) is a sealing ring located in the recess (461).

Optionally, the sleeve (124) is connected to the upper housing (11) of the infusion pump by means of a bolt (413).

Optionally, the lower housing assembly (33) of the infusion pump comprises a lower housing (19) of the infusion pump, a connecting assembly (331), an electrical component (3313) and a plurality of spring needle connectors. The flick needle support (3331) is arranged on the lower housing (19) of the infusion pump. The spring needle connectors are located on the flick needle support (3331). The connecting assembly (331) comprises a plurality of connecting caps, which are connected to the electrical component (3313) respectively. The connecting caps are sleeved on the spring needle connectors.

Optionally, there are three spring needle connectors, which are a first spring needle connector (120), a second spring needle connector (121), and a third spring needle connector (122) respectively.

Optionally, the lower housing (19) of the infusion pump is provided with a first charging port (311) and a second charging port (312), wherein the first charging port (311) and the second charging port (312) are located on the outer side of the flick needle support (3331); the first charging port (311) and the second charging port (312) are opposite to the first spring needle connector (120) and the third spring needle connector (122) respectively; the first charging port (311) and the second charging port (312) are used to charge the rechargeable battery (14).

Optionally, there are two connecting caps, which are a first connecting cap (118) and a second connecting cap (119) respectively.

Optionally, the lower housing (19) of the infusion pump is provided with a waterproof breathable membrane (17), a vibration motor (16), a first connecting cap (118), a second connecting cap (119), a first spring needle connector (120), a second spring needle connector (121), a third spring needle connector (122), a connector pressing block (110), and a restart switch (132), wherein the vibration motor (16) is connected to the first connecting cap (118) and the second connecting cap (119) respectively by means of wires; the first connecting cap (118) and the second connecting cap (119) are sleeved on the spring needle connectors respectively.

Optionally, the first connecting cap (118), the second connecting cap (119) and the spring needle connectors which are not sleeved with the connecting caps abut against the circuit board (36) respectively. Thus, the vibration motor is connected to the printed circuit board of the upper housing assembly of the infusion pump by means of the spring needle connectors, thereby avoiding the welding process and making assembly more convenient.

The infusion device of the present invention comprises three spring needle connectors, wherein the first spring needle connector and the third spring needle connector can be used for charging batteries, and the spring needle connector sleeved with a connecting cap supplies power to the electrical component. Therefore, the three spring needle connectors complete the above-mentioned two functions and reduce the space occupied by the circuit.

Optionally, the lower housing (19) of the infusion pump is provided with a plurality of air-permeable apertures (4412), and the waterproof breathable membrane (17) covers the air-permeable apertures (4412).

Optionally, the first spring needle connector (120), the first connecting cap (118), and the second connecting cap (119) abut against the printed circuit board (13) respectively.

Optionally, the gearbox assembly (15) comprises a motor (151) and a reduction gear set (152), and the reduction gear set (152) is connected to the transmission assembly.

Optionally, the reduction gear set (152) is connected to the screw rod (127).

Optionally, a gear reduction ratio of the reduction gear set (152) is between 90 and 110, and the screw rod is between M2 and M3.

The second aspect of the present invention further provides an assembly method for an infusion device, applied to the infusion device according to the first aspect of the present invention, characterized in that the track (114) of the infusion pump and the guide rail (115) of the liquid storage device (112) are aligned and snapped, and pushed against each other;
when the track almost reaches the bottom, the hook (116) on the infusion pump and the pin (1130) of the liquid storage device (112) are snapped, so that the liquid storage device (112) and the infusion pump are assembled into an infusion device.

Optionally, the assembly method comprises the step of connecting spring needle connectors, which step specifically comprises:
welding the first connecting cap (118) and the second connecting cap (119) together with an outlet terminal of the vibration motor (16) by means of wires;
adhering the vibration motor (16) to the lower housing (19) of the infusion pump;
then sleeving the first connecting cap (118) and the second connecting cap (119) on the spring needle connectors respectively;
connecting the lower housing (19) of the infusion pump and the upper housing (11) of the infusion pump by using screws; and
causing the first spring needle connector (120), the first connecting cap (118) and the second connecting cap (119) to abut against the printed circuit board (13) of the upper housing assembly of the infusion pump respectively.

The third aspect of the present invention provides use of the infusion device according to the first aspect of the present invention for infusing a drug to a patient.

Optionally, the infusion device is used to infuse insulin to a diabetic patient.

The infusion device of the present invention is assembled in a modular manner, is easy to assemble and maintain, and has excellent waterproof performance, which can reach the waterproof IPX8 level.

### Brief Description of the Drawings

For the purpose of illustration rather than limitation, the present invention will now be described according to the preferred embodiments of the present invention, in particular with reference to the accompanying drawings, in which:
FIG. 1 is a structural schematic diagram of an infusion pump;
FIG. 2 is an exploded view of an infusion pump according to an embodiment of the present invention;
FIG. 3 is a front view of an infusion pump according to an embodiment of the present invention;
FIG. 4 is a back view of an infusion pump according to an embodiment of the present invention;
FIG. 5 is a structural schematic diagram of a liquid storage device according to an embodiment of the present invention;
FIG.6 is a structural schematic diagram of an infusion pump according to an embodiment of the present invention;
FIG. 7 is a structural schematic diagram of a gearbox assembly according to an embodiment of the present invention;
FIG. 8 is a structural schematic diagram of a lower housing assembly of an infusion pump according to an embodiment of the present invention;
FIG. 9 is a structural schematic diagram of an upper housing assembly of an infusion pump according to an embodiment of the present invention;
FIG.10 is an exploded view of an upper housing assembly of an infusion pump according to an embodiment of the present invention;
FIG. 11 is a structural schematic diagram of a transmission mechanism in an upper housing assembly of an infusion pump according to an embodiment of the present invention;
FIG.12 is a structural schematic diagram of a transmission mechanism according to an embodiment of the present invention;
FIG. 13 is a perspective view of a liquid storage device in an embodiment of the present invention from another perspective;
FIG.14 is a perspective view of an infusion pump in an embodiment of the present invention from another perspective;
FIG. 15 is a structural schematic diagram of a side buckle in an embodiment of the present invention;
FIG. 16 is a schematic diagram of a snap-in process of a hook in an embodiment of the present invention;
FIG. 17 is a schematic diagram of the finished snap-in of the hook in an embodiment of the present invention;
FIG. 18 is a structural schematic diagram of a top buckle in an embodiment of the present invention;
FIG. 19 is a schematic diagram of a snap-in process of a bottom plate buckle in an embodiment of the present invention;
FIG. 20 is a schematic diagram of the finished snap-in of the bottom plate buckle in an embodiment of the present invention;
FIG. 21 is a structural schematic diagram of a snap-fit connection of a shell of a liquid storage device in an embodiment of the present invention;
FIG. 22 is a cross-sectional view sectioning a plane perpendicular to an upper surface of a shell of a liquid storage device of FIG. 12along the center of a spring;
FIG. 23 is a structural schematic diagram of a shell of a liquid storage device in an embodiment of the present invention;
FIG. 24 is a schematic diagram of an initial state in which a liquid storage device and an infusion pump that have been mounted are mounted with a bottom plate in an embodiment of the present invention;
FIG. 25 is a structural schematic diagram of a pump bottom groove portion in an embodiment of the present invention;
FIG.26 is a structural schematic diagram of a bottom plate protrusion in an embodiment of the present invention;
FIG. 27 is a structural schematic diagram of the connection of a liquid storage device and an infusion pump that have been mounted to one end of a bottom plate in an embodiment of the present invention;
FIG. 28 is an exploded view of a lower housing assembly of a medical infusion device in an embodiment of the present invention;
FIG. 29 is a back view of a lower housing assembly of a medical infusion device in an embodiment of the present invention;
FIG. 30 is a structural schematic diagram of a lower housing assembling body in an embodiment of the present invention;
FIG. 31 is an exploded view of a lower housing assembling body in an embodiment of the present invention;
FIG. 32 is a structural schematic diagram of a lower housing assembling body in an embodiment of the present invention from another perspective;
FIG. 33 is a structural schematic diagram of a track clamping connection process of the infusion pump according to an embodiment of the present invention;
FIG. 34 is a structural schematic diagram of the finished track clamping connection process of the infusion pump according to an embodiment of the present invention;
FIG.35 is a partial cross-sectional view of an infusion pump according to an embodiment of the present invention;
FIG. 36 is an exploded view of an infusion pump in an embodiment of the present invention;
FIG. 37 is a sectional view of the connection between an upper housing and a lower housing of an infusion pump in an embodiment of the present invention;
FIG. 38 is a structural schematic diagram of a sleeve in an embodiment of the present invention;
FIG. 39 is a schematic diagram of the mounting of a sleeve in an embodiment of the present invention;
FIG. 40 is a schematic diagram of the connection of a sleeve in an embodiment of the present invention;
FIG. 41 is an exploded view of a liquid storage device in an embodiment of the present invention;
FIG. 42 is a structural schematic diagram of a liquid storage device in an embodiment of the present invention;
FIG. 43 is a structural schematic diagram of piston sealing in an embodiment of the present invention;
FIG. 44 is a structural schematic diagram of a lower housing assembly of an infusion pump in an embodiment of the present invention;
FIG. 45 is a back view of a lower housing assembly of an infusion pump in an embodiment of the present invention;
FIG. 46 is a top view of a liquid storage device in an embodiment of the present invention;
FIG. 47 is a perspective view of a liquid storage device in an embodiment of the present invention from another perspective;
FIG. 48 is a front view of a liquid storage device in an embodiment of the present invention;
FIG. 49 is a top view of an infusion pump in an embodiment of the present invention;
FIG. 50 is a front view of an infusion pump in an embodiment of the present invention;
FIG. 51 is a structural schematic diagram of the slide rail connection of an infusion pump in an embodiment of the present invention;
FIG. 52 is a structural schematic diagram of a track clamping portion of an infusion pump in an embodiment of the present invention;
FIG. 53 is a structural schematic diagram of an infusion pump when snapping starts in the embodiment of the present invention;
FIG. 54 is a structural schematic diagram of an infusion pump when snapping is in an intermediate state in an embodiment of the present invention;
FIG. 55 is a structural schematic diagram of an infusion pump when snapping is finished in an embodiment of the present invention.

In the figures, 11 upper housing of the infusion pump; 12 antenna; 13 printed circuit board; 14 rechargeable battery; 15 gear box assembly; 151 motor; 152 reduction gear set; 16 vibration motor; 17 waterproof breathable membrane; 18 assembling screw; 19 lower housing of the infusion pump; 110 connector pressing block; 111 piston; 112 liquid storage device; 113 buzzer; 114 track; 115 guide rail; 116 hook; 117 first sealing member; 118 first connecting cap; 119 second connecting cap; 120 first spring needle connector; 121 second spring needle connector; 122 third spring needle connector; 124 sleeve; 125 second seal; 126 nut push rod; 127 screw rod; 128 contact switch; 129 buckle; 130 pressure sensor; 131 infusion needle; 132 restart switch; 40 infusion pump; 1111 upper top surface of the liquid storage device; 1112 first L-shaped side surface of the liquid storage device; 1113 long side surface of the liquid storage device; 1114 second L-shaped side surface of the liquid storage device; 1115 bottom surface of the liquid storage device; 1116 middle side surface of the liquid storage device ; 1117 lower top surface of the liquid storage device; 1118 short side surface of the liquid storage device; 1121 upper top surface of the infusion pump; 1122 first L-shaped side surface of the infusion pump; 1123 long side surface of the infusion pump; 1124 second L-shaped side surface of the infusion pump; 1125 bottom surface of the infusion pump; 1126 middle side surface of the infusion pump; 1127 lower top surface of the infusion pump; 1128 short side surface of the infusion pump; 1130 pin; 1131 side push button; 1132 bayonet;
26 spring; 28 buckle inlet; 29 assembling body of the liquid storage device and the infusion pump; 210 bottom plate; 235 pump bottom groove; 236 top buckle; 2361 top push button; 2362 spring frame; 2363 claw; 23631 inclined surface; 237 bottom plate buckle; 238 side buckle; 2383 connecting rod; 23821 slope; 239 bottom plate protrusion; 240 shell of the liquid storage device; 2401 long slot;
311 first charging port; 312 second charging port; 32 upper housing assembly of the infusion pump; 324 transmission assembly; 33 lower housing assembly of the infusion pump; 331 connecting assembly; 3313 electrical component; 3331 flick needle support; 35 an assembling body of spring needle connectors with the lower housing of the infusion pump;
4411 butt-jointed surface; 4412 air-permeable aperture; 45 second sealing member; 461 recess; 471 arc-shaped portion; 48 third sealing member; 49 fourth sealing member;
471 arc-shaped portion; 48 third sealing member; 49 fourth sealing member;411 inner wall on the inner side of the opening in the bottom surface of the liquid storage device ; 413 bolt; 416 sleeve mounting hole;
531 limiting member ; 532 first edge; 533 groove; 541 protrusion; 542 second edge; 543 projecting portion.

### Detailed Description of the Invention

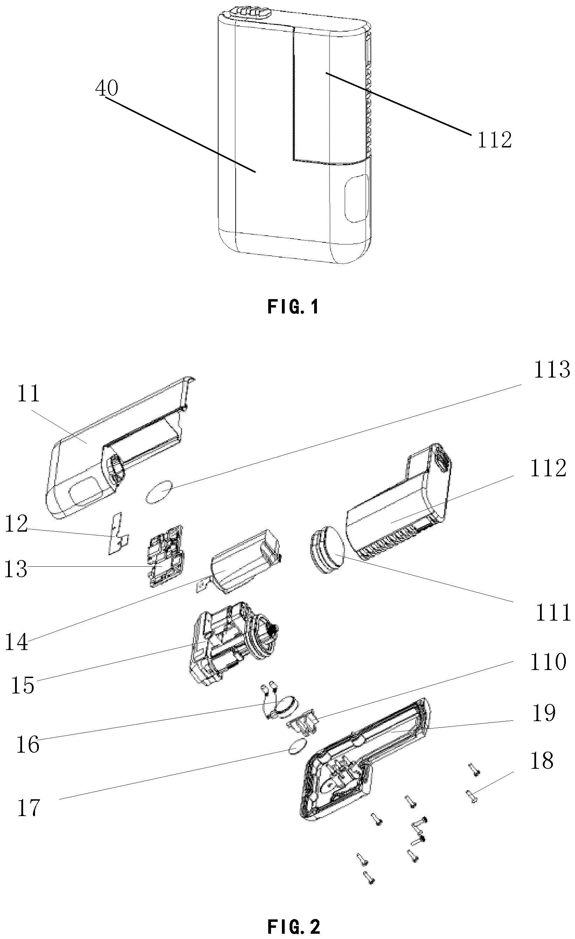

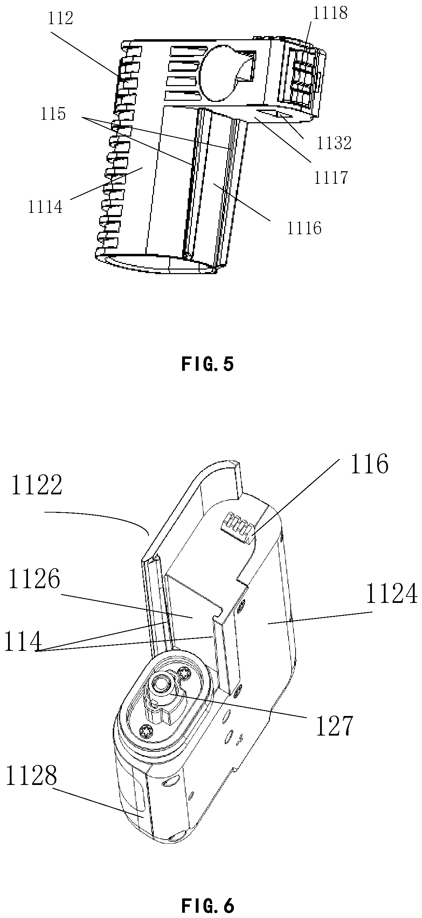

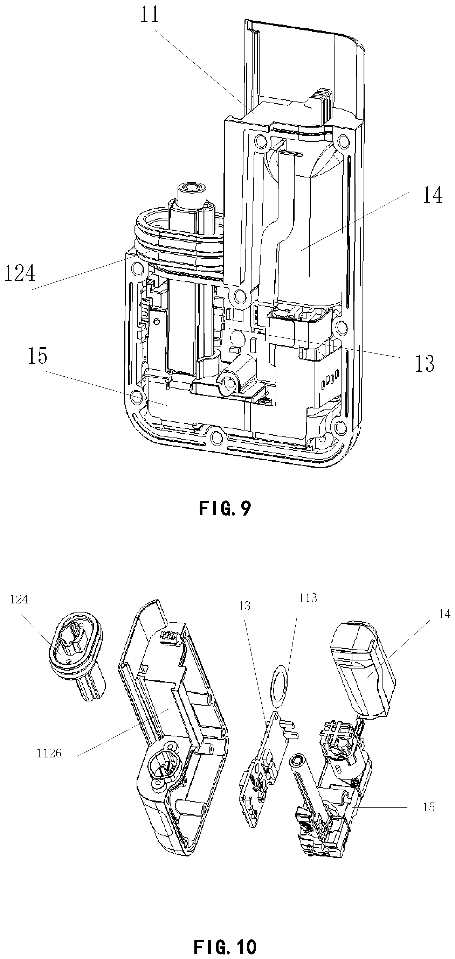

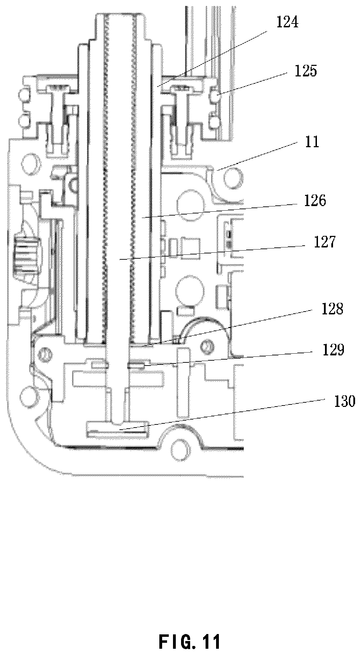

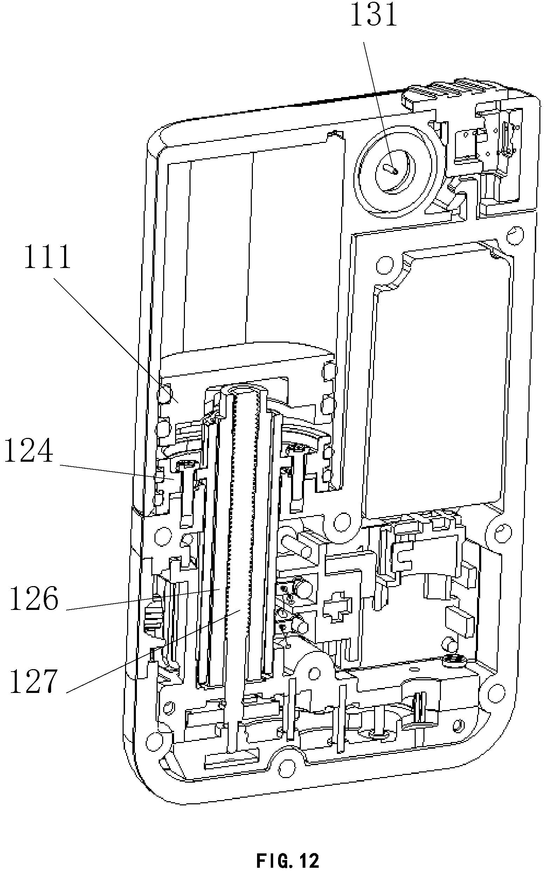

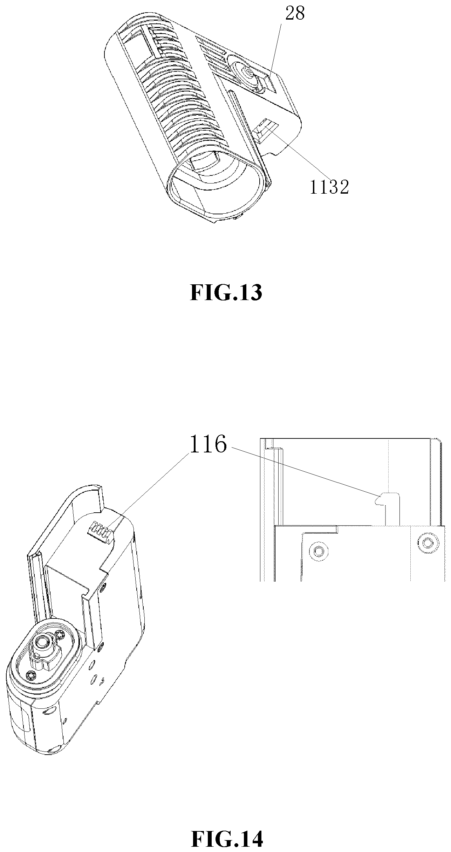

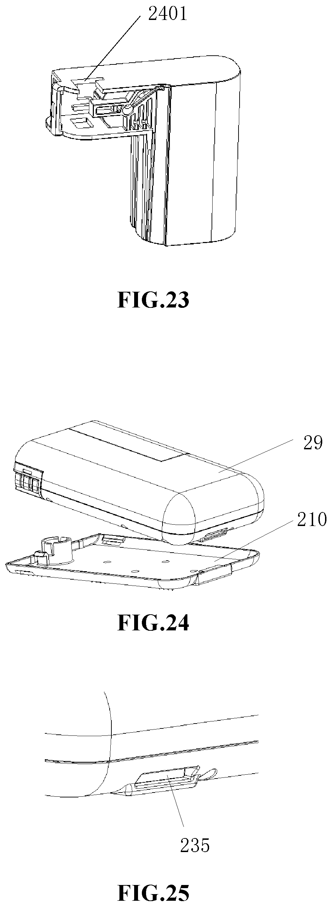

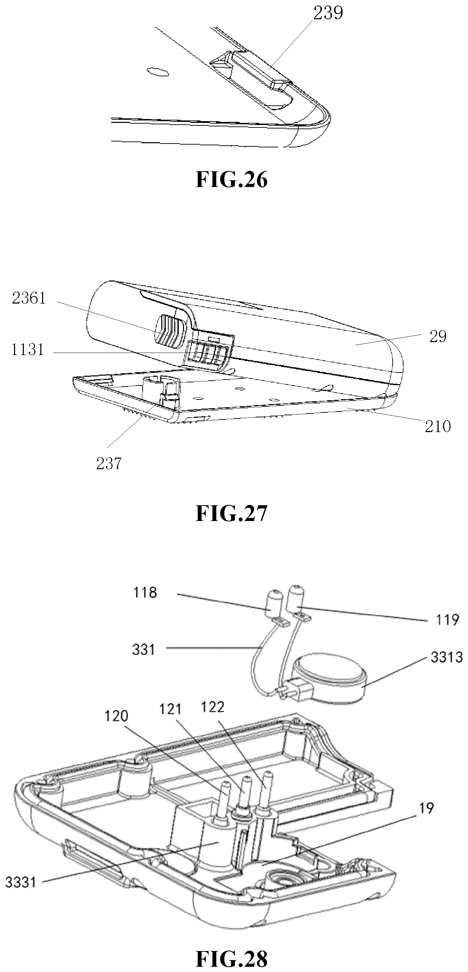

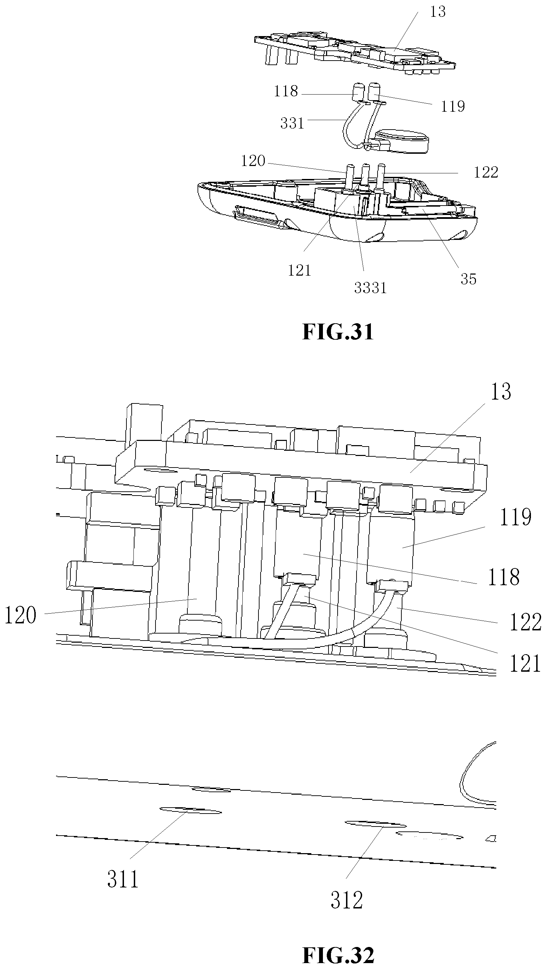

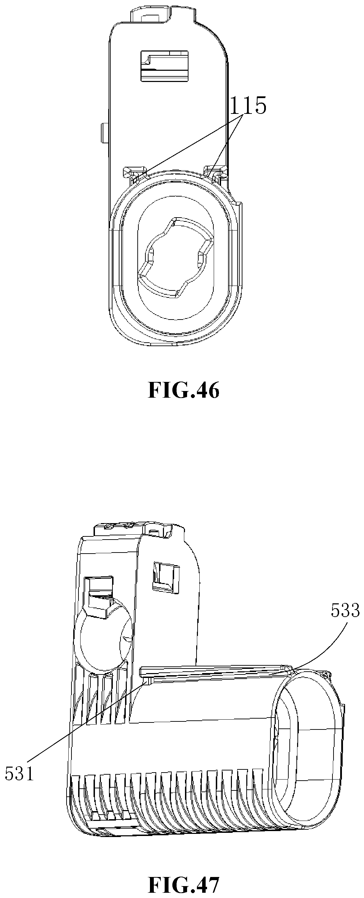

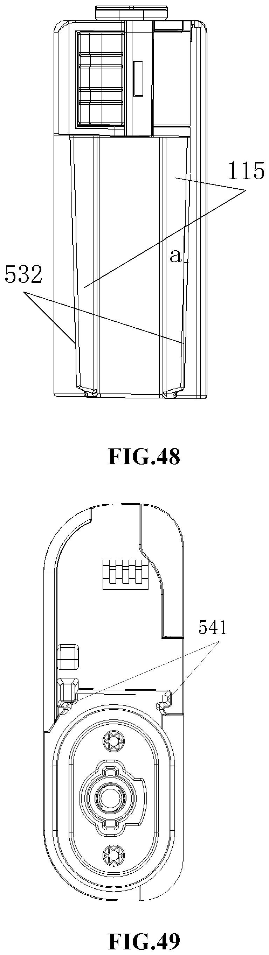

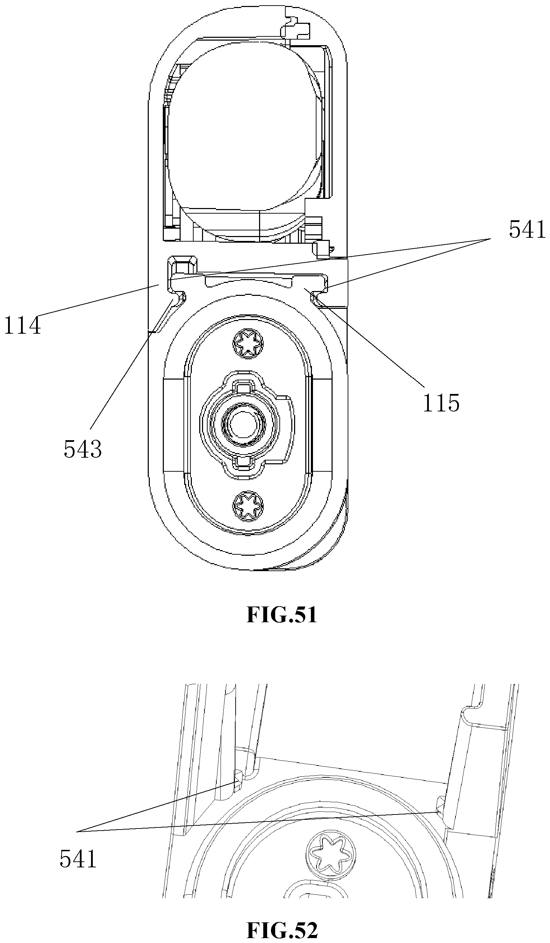

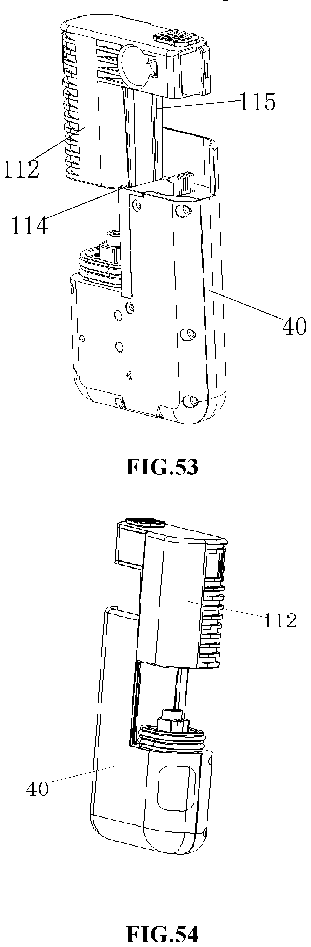

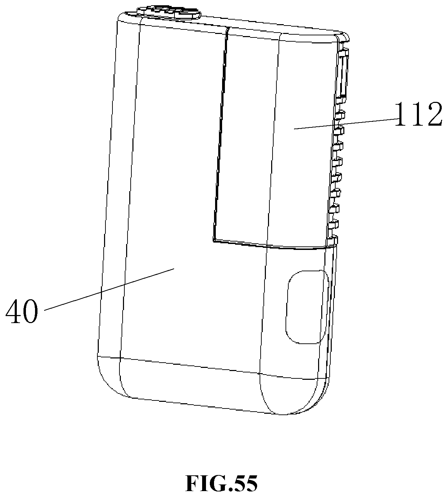

In embodiments of the present invention, the liquid storage device and the infusion pump are connected in a snap-fit manner. Each component is produced in a modular manner, is convenient to mount and dismount, and has good sealing performance, which is described and explained in detail below with reference to FIGS. 1-55.

In embodiments of the present invention, the infusion pump comprises a liquid storage device 112 and an infusion pump 40, and the liquid storage device 112 and the infusion pump 40 are L-shaped.

A first L-shaped side surface 1112 of the liquid storage device, a long side surface 1113 of the liquid storage device, a second L-shaped side surface 1114 of the liquid storage device, and a short side surface 1118 of the liquid storage device constitute the side surfaces of the liquid storage device 112. A middle side surface 1116 of the liquid storage device is located between the long side surface 1113 of the liquid storage device and the short side surface 1118 of the liquid storage device. A bottom surface 1115 of the liquid storage device and a lower top surface 1117 of the liquid storage device are adjacent to the middle side surface 1116 of the liquid storage device. The lower top surface 1117 of the liquid storage device is located between the bottom surface 1115 of the liquid storage device and the upper top surface 1121 of the liquid storage device.

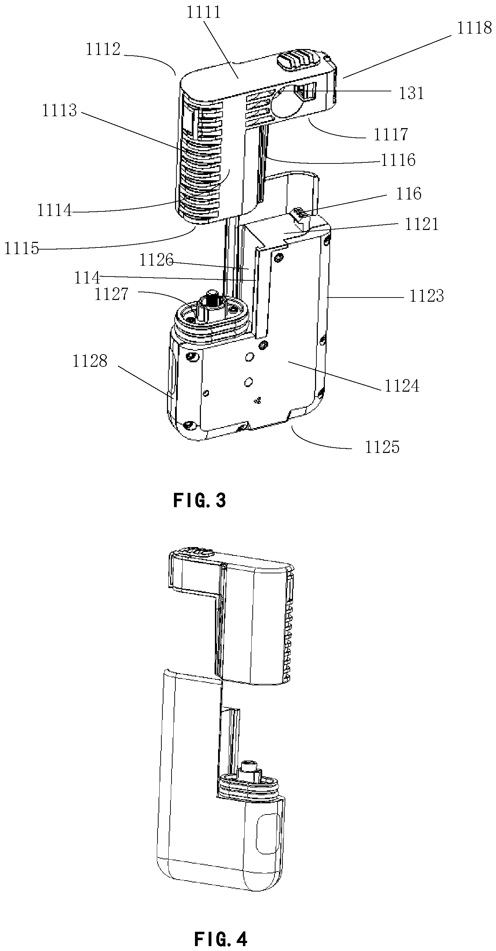

A first L-shaped side surface 1122 of the infusion pump, a long side surface 1123 of the infusion pump, a second L-shaped side surface 1124 of the infusion pump, and a short side surface 1128 of the infusion pump constitute the side surfaces of the infusion pump 40. A middle side surface 1126 of the infusion pump is located between the long side surface 1123 of the infusion pump and the short side surface 1128 of the infusion pump. An upper top surface 1121 of the infusion pump and a low top surface 1127 of the infusion pump are adjacent to the middle side surface 1126 of the infusion pump. The low top surface 1127 of the infusion pump is located between the upper top surface 1121 of the infusion pump and the bottom surface 1125 of the infusion pump. As shown in FIG. 3, the infusion pump is connected to the liquid storage device by means of a slide rail connection structure. Guide rails 115 are provided on the middle side surface 1116 of the liquid storage device, and tracks 114 are provided on the middle side surface 1126 of the infusion pump, wherein the guide rail 115 and the track 114 are engaged with each other so that they can move relative to each other.

The guide rail 115 extends from the bottom surface 1115 of the liquid storage device to the lower top surface 1117 of the liquid storage device. The track 114 extends from the upper top surface 1121 of the infusion pump to the lower top surface 1127 of the infusion pump. The guide rail 115 is arc-shaped and has a first edge 532. When the guide rail 115 slides relative to the track 114, the first edge 532 fits the track 114. The angle between the first edge 532 and the straight line perpendicular to the high top surface 1111 of the liquid storage device is 0.5°-5°, preferably 1.5°.

A limiting member 531 is provided at the end of the guide rail 115 that is close to the lower top surface 1117 of the liquid storage device. The limiting member531 is located in a groove 533 between the guide rail 115 and the middle side surface 1116 of the liquid storage device.

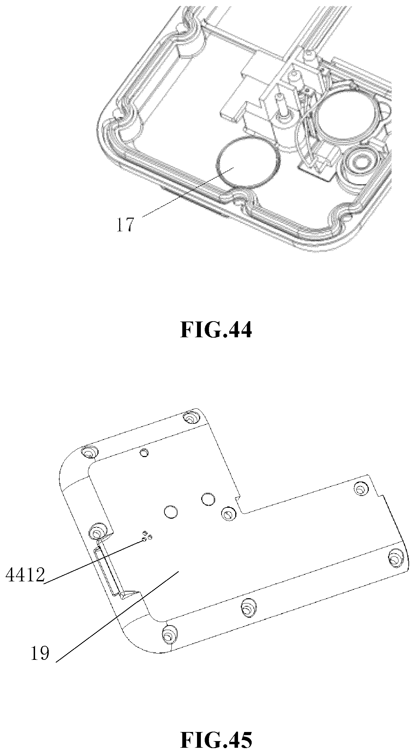

The guide rail 115 of the liquid storage device 112 is used to fix the liquid storage device 112 and the infusion pump40.FIG. 44 is a front view of the liquid storage device, from which it can be seen that the angle a between two guide rails 115 of the liquid storage device (the contact surface with the infusion pump) and the vertical direction is 0.5°-5°, preferably 1.5°. The angle a within this range can facilitate the mutual assembly of the liquid storage device and the infusion pump. The guide rail 115 of the liquid storage device 112 is provided with a limiting member 531 at the bottom which is the position where the guide rail 115 is close to the lower top surface 1117 of the liquid storage device. When the liquid storage device 112 and the infusion pump40 are assembled, the liquid storage device 112 moves downward along the track 114 of the infusion pump40, and when the liquid storage device 112 reaches the bottom, the limiting member 531 blocks the liquid storage device 112from continuing to move toward each other.

The track 114 is provided with a projecting portion 543 stretching inward. The track 114 has a second edge 542. When the track 114 slides relative to the guide rail 115, the second edge 542 fits the guide rail 115.The angle between the second edge 542 and the vertical direction is 0.5°-5°, preferably 0.9°.

The end of the track 114 that is close to the lower top surface 1127 of the infusion pump is provided with a clamping member, which clamps the track 114 and the guide rail 115 at the bottom of the track 114.The clamping member is a protrusion 541 arranged on the rail 114.

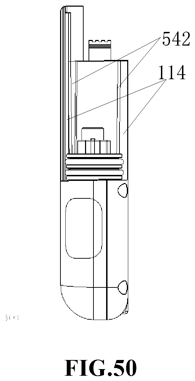

The track 114 of the infusion pump40 is used to fix the liquid storage device 112 and the infusion pump40. FIG. 50 is a front view of the infusion pump, from which it can be seen that the angle a between two tracks 114 of the infusion pump40 (the contact surface with the liquid storage device) and the center line of the middle side surface 1126 of the infusion pump is preferably 0.9 degrees. The angle within this range facilitates the mutual assembly of the liquid storage device 112 and the infusion pump40.When the liquid storage device 112 and the infusion pump40 are assembled, the liquid storage device112 moves downward along the infusion pump40, and the connection between the infusion pump40 and the liquid storage device 112 becomes tighter and tighter. The position of the track 114 of the infusion pump40 that is close to the lower top surface 1127 of the infusion pump is provided with a clamping member. When the track almost reaches the end, the clamping member is clamped with a contact curved surface of the liquid storage device 112.

The contact surface between the guide rail 115 of the liquid storage device and the track 114 of the infusion pump is a curved surface. To ensure easy insertion, only partial sections of the curved surface can be in contact during the insertion process. A clamping member is provided at the bottom of the non-contacting section of the track 114 of the infusion pump40, and the "bottom" is the position of the track 114 that is close to the lower top surface 1127 of the infusion pump. When the track almost reaches the bottom, the clamping member contacts the guide rail 115 of the liquid storage device 112 and is clamped with the guide rail 115, thereby being firmly clamped and uneasy to disengage.

In embodiments of the present invention, in the initial state of the infusion pump, the sleeve is in the bottommost state. During assembly, two guide rails of a drug storage device are aligned with the tracks of the infusion pump and are pushed from top to bottom, so that the guide rails 115 and the tracks 114 are clamped.

According to embodiments of the present invention, when the liquid storage device and the infusion pump are assembled, two guide rails 115 of the liquid storage device 112 and the tracks 114 of the infusion pump are aligned and pushed against each other, so that they are engaged. In embodiments of the present invention, the infusion device is quick and convenient to assemble.

The upper top surface 1121 of the infusion pump is connected to the low top surface 1117 of the liquid storage device by means of a first snap-fit structure. The first snap-fit structure comprises a hook 116 arranged on the upper top surface 1121 of the infusion pump and a bayonet 1132 arranged in the low top surface 1117 of the liquid storage device, wherein the hook 116 is opposite to the bayonet1132. A pin 1130 for snapping with the hook 116 is provided on the inner side of thebayonet1132. When the hook 116 enters the bayonet1132, the hook 116 can push the pin 1130 to one side. When the hook 116 crosses the pin 1130, the pin 1130 moving to one side can move to the other direction under the action of the restoring force, so that the hook 116 and the pin 1130are hooked.

The hook 116 is arranged on the upper top surface 1121 of the infusion pump, and thebayonet1132 is arranged on the low top surface 1117 of the liquid storage device, wherein the hook116 is opposite to the bayonet 1132.Thepin 1130 for snapping with the hook116 is arranged on the inner side of the bayonet 1132, and the pin 1130 is connected to a side push button 1131 arranged on the shell of the liquid storage device.

The pin1130 is connected to a connecting rod 2383, which is connected to the side push button 1131 arranged on the shell 240 of the liquid storage device. The inner side of the side push button 1131 is provided with a spring 26 abutting against the side push button 1131.

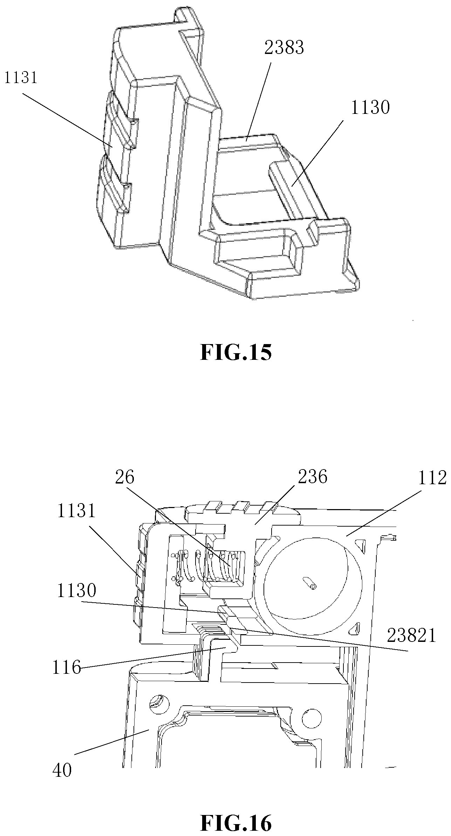

Referring to FIG. 16, one side of the pin 1130 is provided with a slope 23821, which faces the hook 116 and is presented as a line segment in the figure. When the hook116 enters the bayonet1132, the hook116 presses against the slope 23821, so that the pin 1130 moves toward a side away from the hook116, thereby squeezing the spring 26.

In embodiments of the present invention, in the initial state of the infusion pump40, the sleeve 124 is at the bottommost end. Two guide rails 115 of the liquid storage device 112 and the tracks 114 of the infusion pump are aligned and are pushed against each other. When the guide rail almost reaches the bottom position, a side buckle 238 on the liquid storage device 112 is snapped with the hook116 of the infusion pump40, thereby realizing the assembly of the infusion pump40 and the liquid storage device 112.

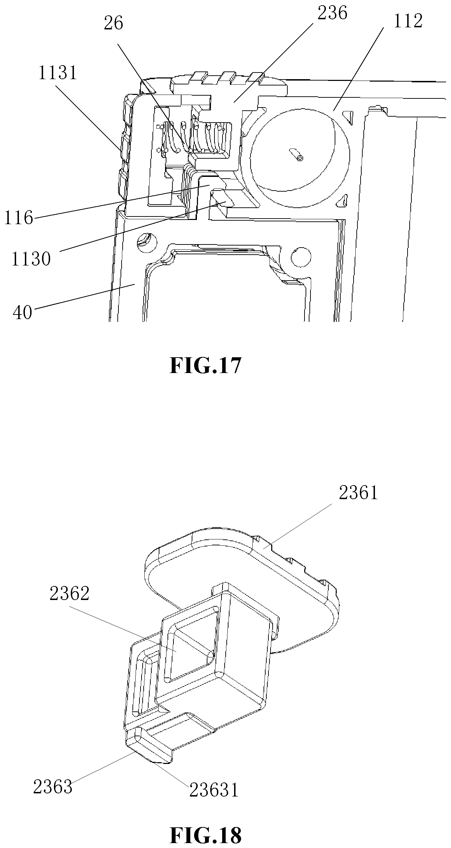

When the liquid storage device 112 and the infusion pump40 are assembled, the hook116 of the infusion pump40 touches the pin1130 of the liquid storage device112 from the bayonet1132 of the liquid storage device 112, as shown in FIG.16. The side buckle 238 moves to the right side. When the hook116 of the infusion pump passes over the pin 1130 of the liquid storage device 112, the pin 1130 moving to the right side moves to the left due to the elastic force of the spring 26, so that the hook116 and the pin 1130 are hooked, as shown in FIG.17, to achieve the installation of the liquid storage device 112 and the infusion pump40.

The infusion pump further comprises a bottom plate 210 which is connected to the assembling body 29 of the liquid storage device and the infusion pump.

The bottom plate 210 is provided with a bottom plate buckle 237.The shell 240 of the liquid storage device is provided with a buckle inlet 28, the inner side of which is provided with a top buckle 236.The top buckle 236 is provided with a claw 2363 for snapping with the bottom plate buckle 237. The claw 2363 is connected to a top push button 2361 arranged on the shell 240 of the liquid storage device by means of a connecting member.

The connecting member is a spring frame 2362, which is opposite to the inner side of the side push button 1131.The spring 26 is located between the spring frame 2362 and the side push button 1131.

The top push button 2361 is located on the surface of the shell 240 of the liquid storage device. The shell 240 of the liquid storage device is provided with a long slot 2401 through which the connection part between the top push button 2361 and the spring frame 2362 passes. When the top push button 2361 is pushed, the connecting part can move along the long slot 2401.

One side of the claw 2363 is provided with an inclined surface 23631. When the bottom plate buckle 237 enters the buckle inlet 28, the bottom plate buckle 237 presses against the inclined surface 23631, so that the claw 2363 moves toward one side, thereby squeezing the spring 26.

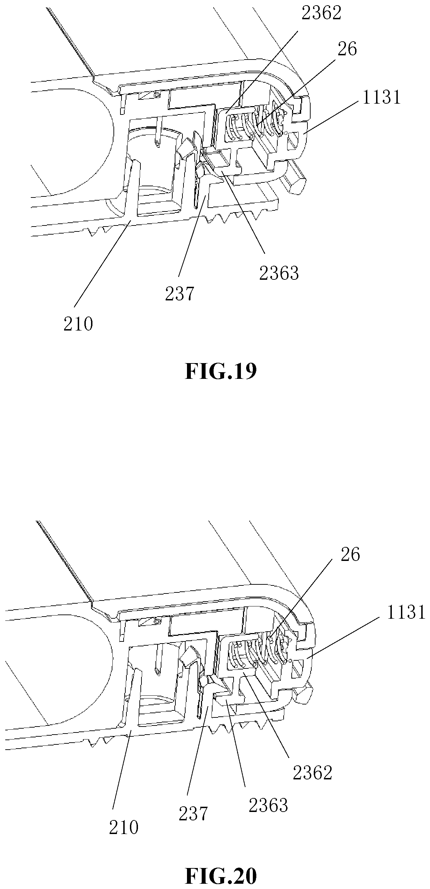

As shown in FIGS. 19 and 20, when the bottom plate buckle 237 on the bottom plate 210 touches the top buckle 236 of the liquid storage device 112, the top buckle 236 moves to the right side. When the bottom plate buckle 237 moves upward and passes over the top buckle 236, the top buckle 236 moves to the left due to the elastic force of the spring and simultaneously snaps the bottom plate buckle 237, thereby realizing the assembly of the assembling body 29 of the liquid storage device and the infusion pump with the bottom plate 210.

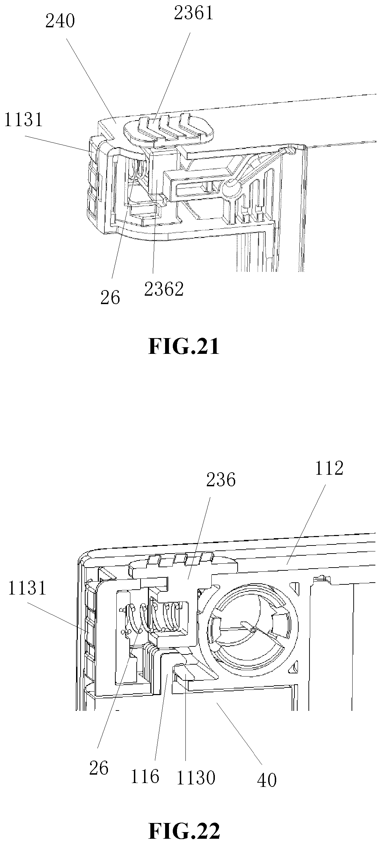

As shown in FIG. 20, when the top buckle 236 on the shell 240of the liquid storage device is pushed to the right (to the left in FIG. 21), the top buckle 236 moves to the right (to the left in FIG. 22), and simultaneously the top buckle 236 is separated from the bottom plate buckle 237 on the bottom plate 210, so that the liquid storage device 112 can be separated from the bottom plate 210, and the assembling body 29 of the liquid storage device and the infusion pump is separated from the bottom plate 210.

As shown in FIG. 22, when the side buckle 238 moves to the right when it is pressed, the pin1130 of the side buckle 238 is disengaged from the hook116, so that the liquid storage device112 can be separated from the infusion pump.

A pump bottom groove 235 is provided at the end on the side of the infusion pump 40 that is close to the bottom plate 210. A bottom plate protrusion 239 is provided at a corresponding position on the bottom plate 210. The bottom plate buckle 237 and the bottom plate protrusion 239 are respectively located at two ends of the bottom plate 210.The bottom plate protrusion 239 can be inserted into the pump bottom groove 235 and can rotate relatively in the pump bottom groove 235.

The pump bottom groove 235 of the infusion pump40 is aligned with the bottom plate protrusion 239, and after they are engaged, the infusion pump40 is pressed toward the direction of the bottom plate 210. When the liquid storage device 112 and the bottom plate 210 are engaged, the assembly of the assembling body 29 of the liquid storage device and the infusion pump with the bottom plate 210 is achieved.

According to embodiments of the present invention, when the liquid storage device112 and the infusion pump40 are assembled, two guide rails 115 of the liquid storage device112 and the tracks 114 of the infusion pump40 are aligned and pushed in opposite directions. At the same time, the side buckle 238 of the liquid storage device112 is snapped with the hook116 of the infusion pump40, so that the liquid storage device and the infusion pump are snapped. The pump bottom groove 235 of the infusion pump40 in the assembling body 29 of the liquid storage device and the infusion pump is aligned with the bottom plate protrusion 239 and is engaged with it, and the infusion pump40 is pressed toward the direction of the bottom plate 210 so as to achieve the assembly of the infusion device. Therefore, the infusion device of the present invention can be assembled quickly and conveniently.

According to embodiments of the present invention, when in use, the top buckle 236 on the shell 240 of the liquid storage device is pushed such that the top buckle 236 is separated from the bottom plate buckle 237, and the assembling body 29 of the liquid storage device and the infusion pump is separated from the bottom plate 210.The side buckle 238 is pressed such that the side buckle 238 is disengaged from the hook116, i.e., the liquid storage device 112 can be separated from the infusion pump40.

In embodiments of the present invention, the liquid storage device moves downward. After the track 114 on the infusion pump and the guide rail 115 of the liquid storage device are snapped, the hook116 on the infusion pump and the pin 1130 of the liquid storage device are snapped, and the liquid storage device 112 and the infusion pump are assembled into an infusion device. When the liquid storage device 112 is replaced, the liquid storage device can be replaced by pressing the side button. In embodiments of the present invention, the liquid storage device and the infusion pump are easy to assemble and disassemble and are convenient to use.

The infusion pump comprises an upper housing assembly 32 and a lower housing assembly 33. The lower housing assembly 33 of the infusion pump comprises a lower housing 19, a connecting assembly 331, an electrical component 3313 and a plurality of spring needle connectors. After removing the liquid storage device 112 along the track direction and detaching the upper housing assembly 32 of the infusion pump, the lower housing assembly 33 of the infusion pump is visible, and the spring needle connectors are on the lower housing assembly 33 of the infusion pump. Pogopin flick needles can be used as spring needle connectors.

The lower housing 19 of the infusion pump is provided with a flick needle support 3331, on which the spring needle connectors are located. The connecting assembly 331 comprises a plurality of connecting caps, which are connected to the electrical component 3313 of the active part respectively. In embodiments of the present invention, the electrical component is a vibration motor, and the connecting caps are sleeved on the spring needle connectors. The spring needle connectors and the lower housing 19 of the infusion pump are assembled first, and then the connecting caps which have been welded with the electrical component 3313 are sleeved on the spring needle connectors to form the lower housing of the infusion pump.

A printed circuit board 13 is provided on the side of the upper housing assembly 32 of the infusion pump that is adjacent to the lower housing assembly33 of the infusion pump. A rechargeable battery 14 is provided to be connected to the printed circuit board 13.

There are three spring needle connectors, namely a first spring needle connector 120, a second spring needle connector 121, and a third spring needle connector 122.

The lower housing 19 of the infusion pump is provided with a first charging port 311 and a second charging port 312, which are located on the outside of the flick needle support 3331.The first charging port 311 and the second charging port 312 are opposite to the first spring needle connector 120 and the third spring needle connector 122respectively.The first charging port 311 and the second charging port 312 are used to charge the rechargeable battery 14.

There are two connecting caps, namely a first connecting cap 118 and a second connecting cap 119. The first connecting cap 118 is sleeved on the second spring needle connector 121.The second connecting cap 119 is sleeved on the third spring needle connector 122.

The first spring needle connector 120, the first connecting cap 118, and the second connecting cap 119 abut against the printed circuit board 13 respectively.

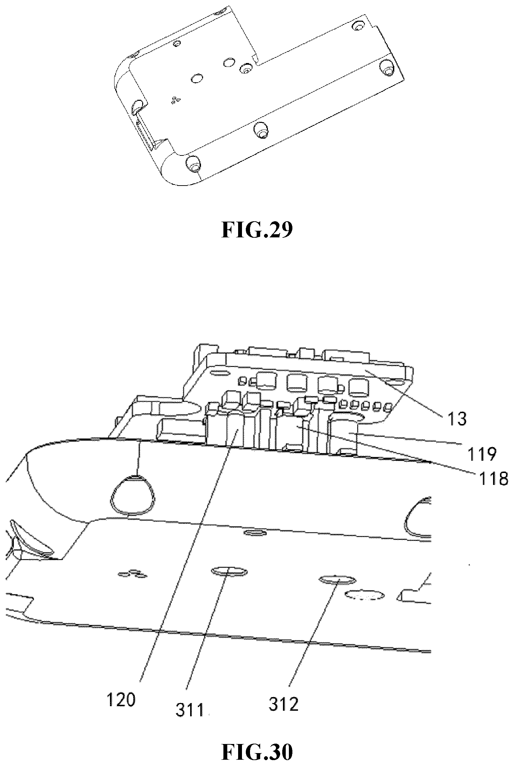

After the spring needle connectors and the lower housing 19 of the infusion pump are assembled, they are installed together with the connecting component 331 and then assembled with the printed circuit board 13 to form a lower housing assembling body in which the circuit board, the connecting caps, the electrical component, the spring needle connectors, and the lower housing of the infusion pump have been assembled. The results are as shown in FIGS. 30-32.

The upper housing assembly 32 of the infusion pump comprises an upper housing 11 of the infusion pump. A sealing ring is provided at the butt-jointed surface between the lower housing 19 of the infusion pump and the upper housing 11 of the infusion pump. The lower housing 19 of the infusion pump and the upper housing 11 of the infusion pump are connected by means of screws.

In embodiments of the present invention, the lower housing of the infusion pump is assembled with the upper housing of the infusion pump by using screws, which is easy to assemble and disassemble, thereby facilitating subsequent maintenance of the infusion device or replacement of batteries.

The upper housing assembly 32 of the infusion pump further comprises a driving gearbox assembly, which comprises a motor 151 and a reduction gear set 152.The motor 151 is electrically connected to the printed circuit board 13. The reduction gear set 152 is connected to the transmission assembly 324.

The motor provides power and drives the transmission assembly by means of the reduction gear set to work. The transmission assembly comprises a screw rod and a nut push rod. The screw rod rotates to push the nut push rod, thereby completing the upward or downward movement of the nut push rod.

In embodiments of the present invention, the rechargeable battery is charged by means of a spring needle connector in a contact-type charging manner, which on the one hand is convenient to charge and on the other hand has good waterproof performance and can reach IPX8 waterproof level.

According to embodiments of the present invention, three spring needle connectors and two connecting caps accomplish two functions, i.e., charging and connecting the electrical component to the circuit board, and the function is accomplished by using three connection lines, which occupies less space and is convenient to assemble.

According to embodiments of the present invention, the connecting cap is directly sleeved on the spring needle connector, so that the spring inside the spring needle connector can be used to squeeze and connect the circuit board, which reduces the welding process and is more convenient to assemble.

As shown in FIGS. 2, 9, 10, 11 and 14, the infusion pump comprises an upper housing assembly of the infusion pump and a lower housing assembly of the infusion pump. The upper housing assembly of the infusion pump comprises an antenna 12, a buzzer 113, a printed circuit board 13, a rechargeable battery 14, a gearbox assembly 15 and a transmission assembly. The upper housing 11 of the infusion pump is connected to the antenna 12, the printed circuit board 13, the rechargeable battery 14, the gearbox assembly 15 and the transmission assembly. The buzzer 113 and the printed circuit board 13 are connected by welding.

In embodiments of the present invention, the antenna 12 is assembled into the upper housing 11 of the infusion pump, including but not limited to, for example, adhering the antenna 12 to the upper housing of the infusion pump with a double-sided tape; welding the buzzer 113 onto the printed circuit board 13; then assembling the buzzer 113 and the printed circuit board 13 to corresponding positions in the upper housing 11 of the infusion pump; and then mounting the rechargeable battery 14 and the gearbox assembly 15 into the upper housing 11 of the infusion pump, thereby assembling into the upper housing assembly of the infusion pump.

The lower housing 19 of the infusion pump comprises a waterproof breathable membrane 17, a vibration motor 16, a first connecting cap 118, a second connecting cap 119, a first spring needle connector 120, a second spring needle connector 121, a third spring needle connector 122, a connector pressing block 110 and a restart switch 132, wherein the vibration motor 16 is connected to the first connecting cap 118 and the second connecting cap 119 respectively by means of wires; and, the first connecting cap 118 and the second connecting cap 119 are sleeved on the spring needle connectors respectively.

In embodiments of the present invention, the waterproof breathable membrane 17 is assembled into the lower housing 19 of the infusion pump, and then the vibration motor 16 is assembled into the lower housing 19 of the infusion pump. The first connecting cap 118 and the second connecting cap 119 are welded together with an outlet terminal of the vibration motor 16 by means of wires, and then the vibration motor 16 is adhered to the lower housing 19 of the infusion pump, and simultaneously the first connecting cap 118 and the second connecting cap 119 are sleeved on the second spring needle connector 121 and the third spring needle connector 122respectively;the connector pressing block 110 and the restart switch 132 are assembled into the lower housing 19 of the infusion pump, thereby assembling the lower housing assembly of the infusion pump. A first sealing member 117 is provided on the butt-jointed surface between the upper housing 11 of the infusion pump and the lower housing 19 of the infusion pump. The lower housing 19 of the infusion pump and the upper housing 11 of the infusion pump are connected by means of screws. The first spring needle connector 120, the first connecting cap 118, and the second connecting cap 119 abut against the printed circuit board 13 respectively.

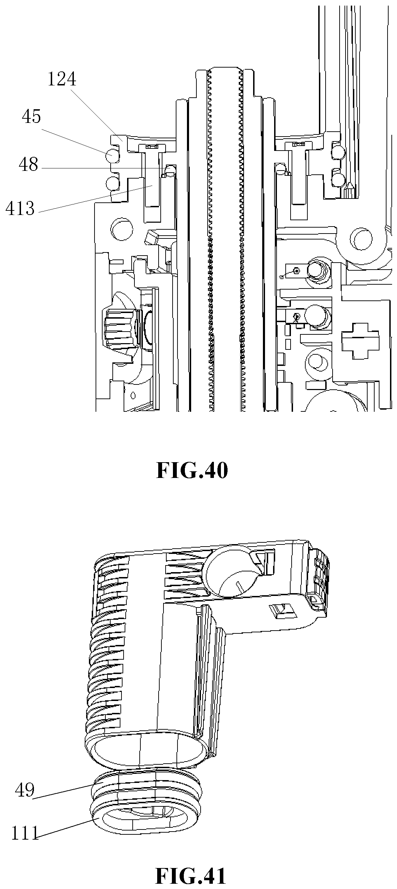

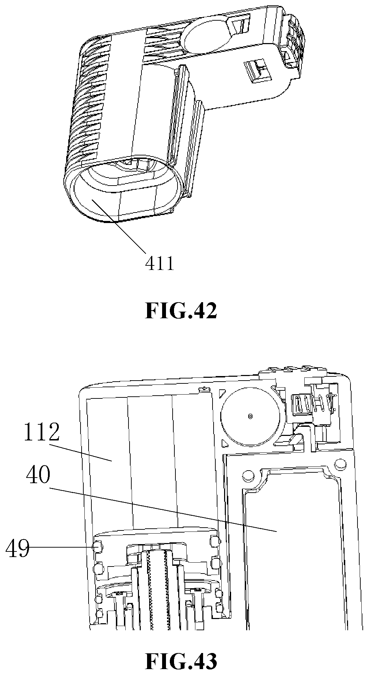

In embodiments of the present invention, the lower housing 19 of the infusion pump is assembled with the upper housing 11 of the infusion pump by using assembling screws 18, and simultaneously the vibration motor and the printed circuit board are assembled in a modular manner, i.e., the vibration motor is connected to the printed circuit board of the upper housing assembly of the infusion pump by means of a spring needle connector. These manners make the assembly and disassembly of the infusion device more convenient. The center of the lower top surface 1127 of the infusion pump is provided with a sleeve 124.The bottom surface 1115 of the liquid storage device is provided with an opening. The lower top surface 1127 of the infusion pump is sealedly connected to the inner wall 411 on the inner side of the opening in the bottom surface of the liquid storage device. FIGS. 41 and 42 show the L-shape of the liquid storage device 112.

The outer side of the sleeve 124 is provided with a second sealing member 45, which is a sealing ring. The sealing connection between the lower top surface 1127 of the infusion pump and the inner wall 411 on the inner side of the opening in the bottom surface of the liquid storage device is achieved by means of the second sealing member 45.

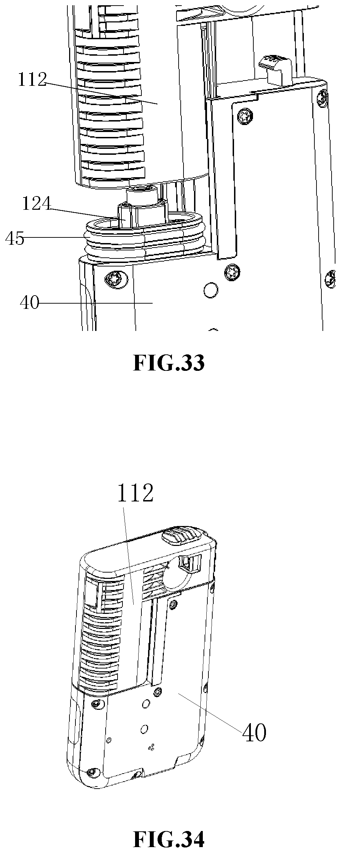

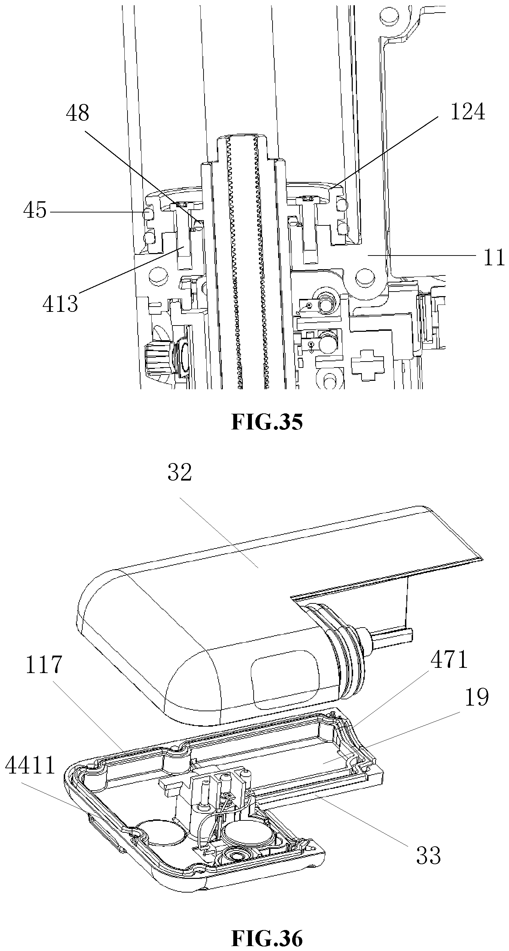

After the guide rail of the liquid storage device and the guide rail of the infusion pump are engaged with each other, the liquid storage device 112 is pushed downward. After the liquid storage device 112reaches the lowest end, the inner wall 411 on the inner side of the opening in the bottom surface of the liquid storage device 112 squeezes the sealing ring, causing the sealing ring to deform, and the assembly is completed, as shown in FIG.34.FIG. 35 is a cross-sectional state of the assembled sealing ring.

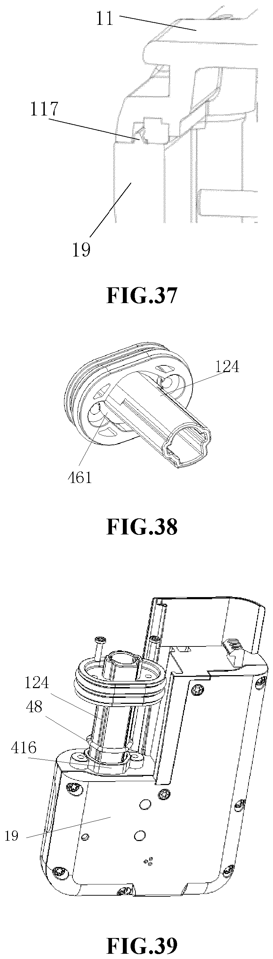

The lower housing 19 of the infusion pump has a butt-joined surface4411 for butt-joining with the upper housing 11 of the infusion pump, and the first sealing member 117 is a sealing ring arranged on the butt-joined surface 4411. The sealing ring is injection molded circumferentially on the butt-joined surface 4411.The butt-joined surface 4411 includes a segment of arc portion 471, which is adapted to the butt-joined surface 4411 of the arc portion 471. The butt-joined surface 4411 is not on a plane, and the butt-joined surface 4411 is not a flat surface, which makes the connection more secure and is also adapted to the inclined angle of the edge of the lower housing. After tightly snapped, the upper and lower housings are fixed with screws. The cross section of the sealing ring is as shown in FIG. 37. The screws cause the upper and lower housings to be squeezed with each other to achieve sealing.

The upper housing 11 of the infusion pump is provided with a sleeve mounting hole 416, and a third sealing member 48 is provided between the sleeve 124 and the upper housing 11 of the infusion pump.

A recess 461 is provided at the portion where the sleeve 124 is connected to the sleeve mounting hole 416, and the third sealing member 48 is a sealing ring located in the recess 461.The sleeve 124 is connected to the upper housing 11 of the infusion pump by means of a bolt 413.The sealing ring is sleeved on the sleeve 124 shown in FIGS. 4-7, and then the sleeve 124 is inserted into a semi-finished product of the infusion pump and installed toward the direction of the bottom surface 1125 of the infusion pump. The sealing ring is assembled in the recess 461 and pressed against the semi-finished product of the infusion pump to complete the assembly, thereby achieving sealing. The cross-sectional view is as shown in FIG.40, from which cross-sectional state of the sealing ring is visible.

The inner side of the opening of the liquid storage device 112 is provided with a piston 111, which is connected to the inner wall 411 on the inner side of the opening in the bottom surface of the liquid storage device by mean of a fourth sealing member 49.The fourth sealing member 49 is a sealing ring arranged on the outer side of the piston 111, which may be an O-ring. The piston 111 with the sealing ring is mounted into the liquid storage device 112. The piston 111 can be pushed by a bolt in the liquid storage device 112 to squeeze out an infusion liquid. The sealing ring is squeezed by the piston and the inner wall 411 on the inner side of the opening in the bottom surface of the liquid storage device and thus is deformed, thereby achieving sealing. The cross-section state of the sealing ring is as shown in FIGS. 4-12.

The lower housing 19 of the infusion pump is provided with a plurality of air-permeable apertures 4412, and the inner side of the lower housing 19 of the infusion pump is provided with a waterproof breathable membrane 17. The waterproof breathable membrane 17 covers the air-permeable apertures 4412.

The waterproof breathable membrane 17 is bonded to the lower housing 19 of the infusion pump by means of its own double-sided adhesive tape. The lower housing of the infusion pump is provided with three small apertures accordingly, through which gas can pass while liquid water is blocked outside.

The infusion device of the present invention has excellent waterproof performance and can be worn during swimming or bathing, and meets the use requirements of patients.

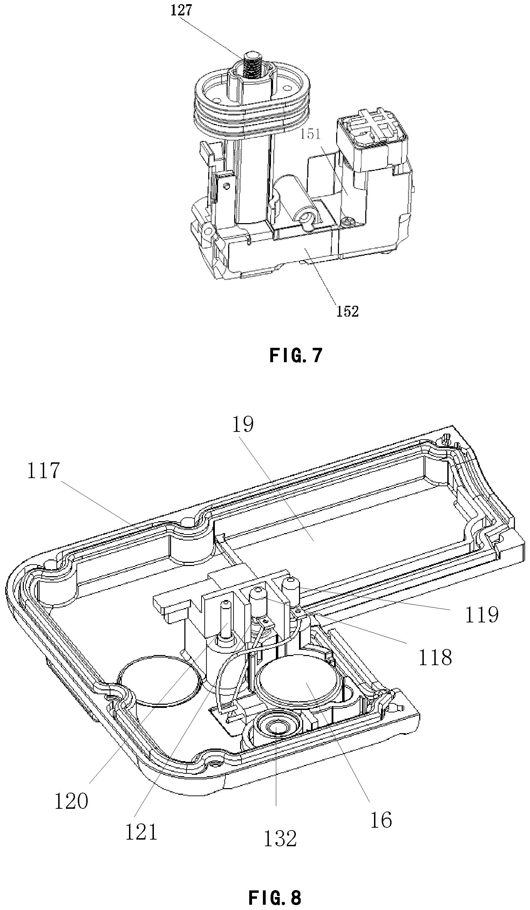

As shown in FIGS. 8, 12 and 13, the transmission assembly comprises a sleeve 124, a screw rod 127, a pressure sensor 130, a nut push rod 126, and a contact switch 128. The sleeve 124 is located on the upper housing 11 of the infusion pump. The nut push rod 126 is arranged in the sleeve 124. The screw rod 127 is located at the center of the nut push rod 126. The screw rod 127 is threadedly connected to the nut push rod 126.The gearbox assembly 15 is connected to the screw rod 127.The pressure sensor 130 is arranged at the bottom of the screw rod 127.The contact switch 128 is arranged at the bottom of the stroke of the nut push rod 126.

The piston 111 is arranged in the liquid storage device 112.An infusion needle 131 is connected to a cavity above the piston 111 by means of a pipeline. The piston 111 is opposite to the nut push rod 126 and is arranged at the top of the nut push rod 126.

In some embodiments of the present invention, while the screw rod 127 rotates clockwise for downward movement, the screw rod 127 can push the nut push rod 126 upward, and the screw rod 127 will touch the pressure sensor 130; the nut push rod 126 will touch the piston 111 and push the piston 111 to move. When the nut push rod 126 touches the piston 111, the pressure of the pressure sensor 130 will increase, triggering an infusion command, and the system will prompt the infusion device to perform the infusion. When the infusion device performs infusion, in the case where the infusion needle 131 is blocked, the pressure of the pressure sensor 130 will reach a threshold, and then the system will prompt the user that a blockage has occurred. When the screw rod 127 rotates counterclockwise, the nut push rod 126 will move back downward, and when it moves to the contact switch 128, a stop command will be triggered, and the screw rod 127 and the nut push rod 126 will stop moving.

In embodiments of the present invention, the second sealing member 125 is mounted inside the sleeve 124.The sleeve 124 is passed through the hole of the upper housing 11 of the infusion pump and then is fixed by using a bolt. The sleeve is assembled with the housing by using a bolt, which can facilitate assembly and disassembly and make the overall structure more compact.

The infusion device of the present invention can be integrally assembled, for example, the rear housing can be disassembled as a whole so as to facilitate subsequent replacement of batteries or maintenance.

The gearbox assembly 15 comprises a motor 151 and a reduction gear set 152, and the reduction gear set 152 is connected to the screw rod 127.In this case, the motor provides power, which drives the screw rod to rotate by means of the reduction gear set; the screw rod pushes the nut push rod, thereby completing the upward or downward movement of the nut push rod.

The infusion device of the present invention can be used to infuse a drug to a patient. Optionally, the infusion device is used to infuse insulin to a diabetic patient.

The above embodiments do not constitute limitations on the protection scope of the present invention. It should be apparent to those skilled in the art that various modifications, combinations, sub-combinations and substitutions may occur depending on design requirements and other factors. Any modification, equivalent substitution, improvement or the like made within the spirit and principles of the present invention should be included in the protection scope of the present invention.

## Claims

**1.** An infusion device, comprising an infusion pump (40) and a liquid storage device (112) connected to the infusion pump, **characterized in that** the liquid storage device (112) and the infusion pump (40) are L-shaped, wherein
the side surfaces of the liquid storage device (112) is composed of a first L-shaped side surface (1112) of the liquid storage device, a long side surface (1113) of the liquid storage device, a second L-shaped side surface (1114) of the liquid storage device, and a short side surface (1118) of the liquid storage device; the middle side surface (1116) of the liquid storage device is located between the long side surface (1113) of the liquid storage device and the short side surface (1118) of the liquid storage device; the bottom surface (1115) of the liquid storage device and the lower top surface (1117) of the liquid storage device are adjacent to the middle side surface (1116) of the liquid storage device; and the lower top surface (1117) of the liquid storage device is located between the bottom surface (1115) of the liquid storage device and the upper top surface (1111) of the liquid storage device;
the side surfaces of the infusion pump (40) is composed of a first L-shaped side surface (1122) of the infusion pump, a long side surface (1123) of the infusion pump, a second L-shaped side surface (1124) of the infusion pump, and a short side surface (1128) of the infusion pump; the middle side surface (1126) of the infusion pump is located between the long side surface (123) of the infusion pump and the short side surface (1128) of the infusion pump; the upper top surface (1121) of the infusion pump and the low top surface (1127) of the infusion pump are adjacent to the middle side surface (1126) of the infusion pump; and the low top surface (1127) of the infusion pump is located between the upper top surface (1121) of the infusion pump and the bottom surface (1125) of the infusion pump.

**2.** The infusion device according to claim 1, **characterized in that** the infusion pump (40) and the liquid storage device (112) are connected by means of a slide rail.

**3.** The infusion device according to claim 1 or 2, **characterized in that**
the middle side surface (1116) of the liquid storage device is provided with guide rails (115) perpendicular to the upper top surface (1111) of the liquid storage device, and the middle side surface (1126) of the infusion pump is provided with tracks (114) perpendicular to the bottom surface (1125) of the infusion pump;
the guide rail (115) and the track (114) are engaged with each other so that they can move relative to each other;
the infusion pump comprises an upper housing assembly and a lower housing assembly;
a first sealing member is provided between the upper housing assembly of the infusion pump and the lower housing assembly of the infusion pump;
the upper housing assembly of the infusion pump and the lower housing assembly of the infusion pump are connected by means of a detachable connecting structure.

**4.** The infusion device according to claim 3, **characterized in that** the guide rail (115) extends from the bottom surface (1115) of the liquid storage device to the lower top surface (1117) of the liquid storage device;
the track (114) extends from the upper top surface (1121) of the infusion pump to the lower top surface (1127) of the infusion pump.

**5.** The infusion device according to claim 3 or 4, **characterized in that** the guide rail (115) has a first edge (532);
when the guide rail (115) slides relative to the track (114), the first edge (532) fits the track (114).

**6.** The infusion device according to claim 5, **characterized in that** there is an angle between the first edge (532) and the center line of the middle side surface (1116) of the liquid storage device, and the angle ranges from 0.5° to 5°.

**7.** The infusion device according to any one of claims 3 to 6, **characterized in that** the end of the guide rail (115) that is close to the lower top surface (1117) of the liquid storage device is provided with a limiting member (531), which is located in a groove (533) between the guide rail (115) and the middle side surface (1116) of the liquid storage device.

**8.** The infusion device according to any one of claims 3 to 7, **characterized in that** the track (114) is provided with a projecting portion (543) stretching inward, and the track (114) is provided with a second edge (542);
when the track (114) slides relative to the guide rail (115), the second edge (542) fits the guide rail (115).

**9.** The infusion device according to claim 8, **characterized in that** there is an angle between the second edge (542) and the center line of the middle side surface (1126) of the infusion pump, and the angle ranges from 0.5° to 5°.

**10.** The infusion device according to any one of claims 3 to 9, **characterized in that** the end of the track (114) that is close to the lower top surface (1127) of the infusion pump is provided with a clamping member, which clamps the track (114) and the guide rail (115) at the bottom of the track (114).

**11.** The infusion device according to claim 10, **characterized in that** the clamping member is a protrusion (541) arranged on the track (114).

**12.** The infusion device according to any one of claims 3 to 11, **characterized in that** the first sealing member is a first sealing member (117);
the upper housing assembly of the infusion pump and the lower housing assembly of the infusion pump are provided with an upper housing (11) of the infusion pump and a lower housing (19) of the infusion pump respectively, and the first sealing member (117) is located on a butt-joined surface between the lower housing (19) of the infusion pump and the upper housing (11)of the infusion pump;
the lower housing (19) of the infusion pump and the upper housing (11) of the infusion pump are connected by means of screws.

**13.** The infusion device according to any one of claims 3 to 12, **characterized in that** the first snap-fit structure comprises a hook (116) arranged on the upper top surface (1121) of the infusion pump and a bayonet (1132) arranged on the lower top surface (1117) of the liquid storage device, wherein
the hook (116) corresponds to the bayonet (1132);
the inner side of the bayonet (1132) is provided with a pin (1130) for snapping with the hook (116), and the pin (1130) is connected to a side push button (1131) arranged on a shell of the liquid storage device.

**13.** The infusion device according to any one of claims 1 to 12, **characterized in that** the upper housing assembly of the infusion pump further comprises an antenna (12), a buzzer (113), a printed circuit board (13), a rechargeable battery (14), a gearbox assembly (15) and a transmission assembly, wherein
the upper housing (11) of the infusion pump is connected to all of the antenna (12), the printed circuit board (13), the rechargeable battery (14), the gearbox assembly (15) and the transmission assembly;
the buzzer (113) is attached to the inner side of the upper housing (11) of the infusion pump and is connected to the printed circuit board (13).

**14.** The infusion device according to claim 13, **characterized in that** the transmission assembly comprises a sleeve (124), a screw rod (127), a pressure sensor (130), a nut push rod (126), and a contact switch (128), wherein
the sleeve (124) is located on the upper housing (11) of the infusion pump;
the nut push rod (126) is arranged in the sleeve (124), the screw rod (127) is located at the center of the nut push rod (126), and the screw rod (127) is threadedly connected to the nut push rod (126);
the gearbox assembly (15) is connected to the screw rod (127);
the pressure sensor (130) is arranged at the bottom of the screw rod (127);
the contact switch (128) is arranged at the bottom of the stroke of the nut push rod (126).

**15.** The infusion device according to claim 14, **characterized in that** a piston (111) is provided in the liquid storage device (112), and an infusion needle (131) is connected to a cavity above the piston (111) by means of a pipeline;
the piston (111) is opposite to the nut push rod (126) and is arranged at the top of the nut push rod (126).

**16.** The infusion device according to any one of claims 12 to 15, **characterized in that** the lower housing (19) of the infusion pump is provided with a waterproof breathable membrane (17), a vibration motor (16), a first connecting cap (118), a second connecting cap (119), a first spring needle connector (120), a second spring needle connector (121), a third spring needle connector (122), a connector pressing block (110), and a restart switch (132), wherein
the vibration motor (16) is connected to the first connecting cap (118) and the second connecting cap (119) respectively by means of wires;
the first connecting cap (118) and the second connecting cap (119) are sleeved on the spring needle connectors respectively.

**17.** The infusion device according to claim 16, **characterized in that** the first spring needle connector (120), the first connecting cap (118), and the second connecting cap (119) abut against the printed circuit board (13) respectively.

**18.** The infusion device according to any one of claims 13 to 17, **characterized in that** the gearbox assembly (15) comprises a motor (151) and a reduction gear set (152), and the reduction gear set (152) is connected to the transmission assembly.

**19.** The infusion device according to claim 18, **characterized in that** the motor (151) is connected to the printed circuit board, and the reduction gear set (152) is connected to the screw rod (127).

**20.** The infusion device according to claim 18 or 19, **characterized in that** a gear reduction ratio of the reduction gear set (152) is between 90 and 110, and the screw rod is between M2 and M3.

**21.** The infusion device according to any one of claims 1 to 20, which is a drug infusion device, preferably an insulin infusion device.

**22.** An assembly method for an infusion device, applied to the infusion device according to claim 17, **characterized in that** the track (114) of the infusion pump and the guide rail (115) of the liquid storage device (112) are aligned and engaged, and pushed against each other;
when the track almost reaches the bottom, the hook (116) on the infusion pump and the pin (1130) of the liquid storage device (112) are snapped, so that the liquid storage device (112) and the infusion pump are assembled into an infusion device.

**23.** The assembly method according to claim 22, **characterized in that** the assembly method comprises the step of connecting spring needle connectors, which step specifically comprises:
welding the first connecting cap (118) and the second connecting cap (119) together with an outlet terminal of the vibration motor (16) by means of wires;
adhering the vibration motor (16) to the lower housing (19) of the infusion pump;
then sleeving the first connecting cap (118) and the second connecting cap (119) on the spring needle connectors respectively;
connecting the lower housing (19) of the infusion pump and the upper housing (11) of the infusion pump by using screws; and
causing the first spring needle connector (120), the first connecting cap (118) and the second connecting cap (119) to abut against the printed circuit board (13) of the upper housing assembly of the infusion pump respectively.

**24.** Use of an infusion device according to any one of claims 1 to 21, for infusing a drug to a patient.

**25.** The use according to claim 24, for infusing insulin to a diabetic patient.
